(19) Europäisches Patentamt
European Patent Office
Office européen des brevets

(11) Veröffentlichungsnummer: **0 013 322**
**A1**

(12) # EUROPÄISCHE PATENTANMELDUNG

(21) Anmeldenummer: **79104681.6**

(22) Anmeldetag: **24.11.79**

(51) Int. Cl.[3]: **C 07 D 285/10**
A 01 N 43/82

(30) Priorität: **07.12.78 DE 2852869**

(43) Veröffentlichungstag der Anmeldung:
**23.07.80 Patentblatt 80/15**

(84) Benannte Vertragsstaaten:
**BE CH DE FR GB IT NL SE**

(71) Anmelder: **BAYER AG**
***Zentralbereich Patente, Marken und Lizenzen***
**D-5090 Leverkusen 1, Bayerwerk(DE)**

(72) Erfinder: **Knops, Hans-Joachim, Dr.**
**Claudiusweg 3**
**D-5600 Wuppertal 1(DE)**

(72) Erfinder: **Brandes, Wilhelm, Dr.**
**Eichendorffstrasse 3**
**D-5653 Leichlingen(DE)**

(72) Erfinder: **Paul, Volker, Dr.**
**Ahornstrasse 5**
**D-5650 Solingen(DE)**

(54) **Neue 3,4-Dioxo-1,2,5-thiadiazolidin-1-oxide (I), ihre Herstellung und Verwendung als Fungizide, die Verbindungen (I) enthaltende fungizide Mittel und deren Herstellung.**

(57) Die neuen 3,4-Dioxo-1,2,5 -thiadiazolidin-1 -oxide der Formel

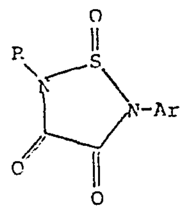

(I)

in welcher Ar für gegebenenfalls substituiertes Aryl steht und R für Alkyl steht, z.B. 2-(3,5 -Dichlorphenyl)-5 -isopropyl -3,4-dioxo-1,2,5 -thiadiazolidin -1-oxid, werden erhalten, wenn man substituierte Schwefeldiimide der Formel R-N=S=N-Ar mit Oxalylchlorid in Gegenwart eines organischen Verdünnungsmittels umsetzt und die dabei zunächst entstehenden 1,1 -Dichlor -3,4-dioxo-1,2,5 -thiadiazolidine ohne deren Isolierung in üblicher Weise zu den entsprechenden 3,4-Dioxo-1,2,5 -thiadiazolidin-1-oxiden hydrolysiert.

Die Wirkstoffe (I) können in Form von Pflanzenschutzmitteln mit besonders gutem Erfolg zur Bekämpfung von Botrytis-Arten verwendet werden.

EP 0 013 322 A1

Croydon Printing Company Ltd.

BAYER AKTIENGESELLSCHAFT
Zentralbereich
Patente, Marken und Lizenzen

5090 Leverkusen, Bayerwerk
Slr/AB
Ia

BEZEICHNUNG GEÄNDERT
siehe Titelseite

3,4-Dioxo-1,2,5-thiadiazolidin-1-oxide, Verfahren zu ihrer Herstellung sowie ihre Verwendung als Fungizide

Die vorliegende Erfindung betrifft neue 3,4-Dioxo-1,2,5-thiadiazolidin-1-oxide, ein Verfahren zu ihrer Herstellung sowie ihre Verwendung als Fungizide.

Es ist bereits bekannt geworden, daß Thiuramdisulfide, wie beispielsweise Tetramethyl-thiuramdisulfid, gute fungizide Eigenschaften aufweisen (vergleiche US-Patentschrift 1 972 961). Ebenfalls ist bereits bekannt, daß Zink-ethylen-1,2-bis-dithiocarbamidat ein gutes Mittel zur Bekämpfung von pilzlichen Pflanzenkrankheiten ist (vergleiche Phytopathology 33, 1113 (1963)). Die Wirkung beider Stoffklassen ist jedoch in bestimmten Indikationsbereichen, insbesondere bei niedrigen Aufwandmengen und -konzentrationen, nicht immer voll befriedigend.

Es wurden nun als neue Verbindungen die 3,4-Dioxo-1,2,5-thiadiazolidin-1-oxide der Formel

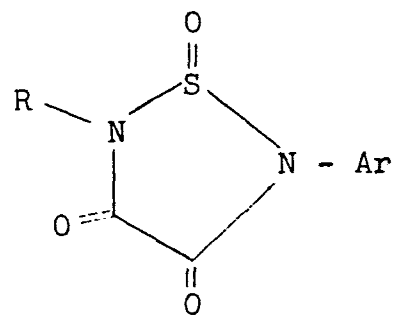

(I)

in welcher

Ar für gegebenenfalls substituiertes Aryl steht und

R für Alkyl steht,

gefunden.

Weiterhin wurde gefunden, daß man die 3,4-Dioxo-1,2,5-thiadiazolidin-1-oxide der Formel (I) erhält, wenn man substituierte Schwefeldiimide der Formel

$$R - N = S = N - Ar \qquad (II)$$

in welcher

Ar und R die oben angegebene Bedeutung haben,

mit Oxalylchlorid der Formel

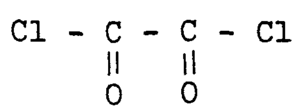

(III)

in Gegenwart eines organischen Verdünnungsmittels umsetzt und die dabei zunächst entstehenden 1,1-Dichlor-3,4-dioxo-1,2,5-thiadiazolidine der Formel

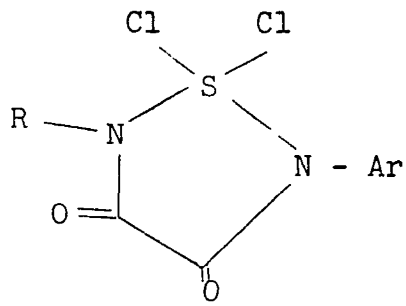

(IV)

in welcher

Ar und R die oben angegebene Bedeutung haben,

ohne deren Isolierung in üblicher Weise zu den entsprechenden 3,4-Dioxo-1,2,5-thiadiazolidin-1-oxiden hydrolysiert.

Außerdem wurde gefunden, daß die 3,4-Dioxo-1,2,5-thiadiazolidin-1-oxide der Formel (I) gute fungizide Eigenschaften aufweisen.

Uebrraschenderweise zeigen die erfindungsgemäßen 3,4-Dioxo-1,2,5-thiadiazolidin-1-oxide eine erheblich höhere fungizide Wirkung, insbesondere gegen Botrytis-Arten, als die aus dem Stand der Technik bekannten Verbindungen Tetramethyl-thiuramdisulfid und Zink-ethylen-1,2-bisdithiocarbamidat, welche anerkannt gute Mittel gleicher Wirkungsrichtung sind. Die erfindungsgemäßen Wirkstoffe stellen somit eine Bereicherung der Technik dar.

Die erfindungsgemäßen 3,4-Dioxo-1,2,5-thiadiazolidin-1-oxide sind durch die Formel (I) allgemein definiert. In der Formel (I) steht Ar vorzugsweise für gegebenenfalls einfach oder mehrfach, gleich oder verschieden substituiertes Phenyl, wobei als Substituenten vorzugsweise infrage kommen: Halogen, Alkyl mit 1 oder 2 Kohlenstoffatomen sowie Halogenalkyl mit bis zu 2 Kohlenstoff- und bis zu 3 gleichen oder verschiedenen Halogenatomen, wobei als Halogenatome vorzugsweise Fluor und Chlor genannt seien. R steht vorzugsweise für geradkettiges oder verzweigtes Alkyl mit 1 bis 6 Kohlenstoffatomen.

Ganz besonders bevorzugt sind diejenigen Verbindungen der Formel (I), in denen Ar für einfach oder zweifach, gleich oder verschieden durch Chlor, Brom, Methyl oder Trifluormethyl substituiertes Phenyl steht und R für Methyl, Ethyl, n-Propyl, iso-Propyl, iso.-Butyl, sek.-Butyl oder tert.-Butyl steht.

Im einzelnen seien außer den bei den Herstellungsbeispielen genannten Verbindungen die folgenden Verbindungen der allgemeinen Formel (I) genannt:

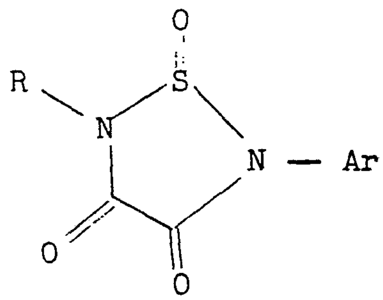

(I)

| R | Ar | R | Ar |
|---|---|---|---|
| $CH_3$ | 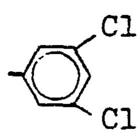 | $C_2H_5$ | 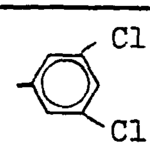 |
| $i-C_3H_7$ | 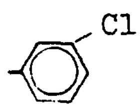 | $CH_3$ | 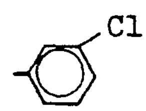 |
| $i-C_3H_7$ | 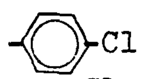 | $CH_3$ | 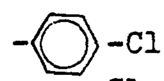 |
| $i-C_3H_7$ | 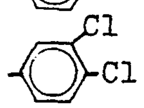 | $CH_3$ | 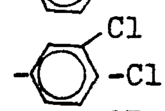 |
| $i-C_3H_7$ | 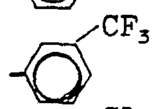 | $CH_3$ | 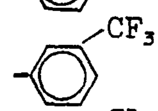 |
| $C_2H_5$ | 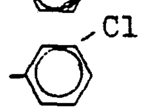 | $C_2H_5$ | 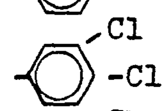 |
| $C_2H_5$ | 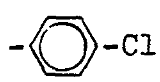 | $C_2H_5$ | 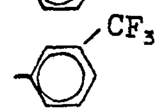 |

Verwendet man beispielsweise N-tert.-Butyl-N'-3,5-dichlorphenyl-schwefeldiimid und Oxalylchlorid als Ausgangsstoffe, so kann der Reaktionsablauf des erfindungsgemäßen Verfahrens durch das folgende Formelschema wiedergegeben werden:

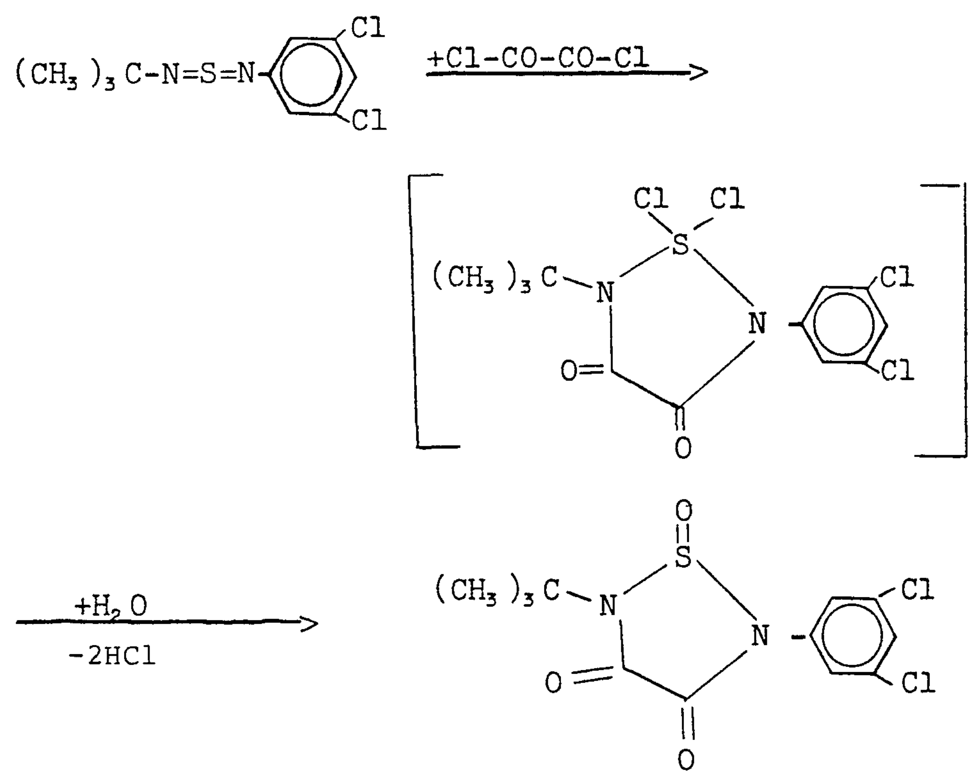


Die bei der Durchführung des erfindungsgemäßen Verfahrens als Ausgangsstoffe benötigten substituierten Schwefeldiimide sind durch die Formel (II) allgemein definiert. In dieser Formel stehen Ar und R vorzugsweise für diejenigen Reste, die bereits im Zusammenhang mit der Formel (I) vorzugsweise für die Reste genannt wurden.

Die substituierten Schwefeldiimide der Formel (II) sind teilweise bekannt (vergleiche Synthesis 1977, 63). Noch nicht bekannte lassen sich in allgemein bekannter Art und Weise herstellen, indem man aliphatische Schwefeldiimide der Formel

$$R - N = S = N - R \qquad (V)$$

in welcher

R die oben angegebene Bedeutung hat,

mit Isocyanaten der Formel

$$Ar - N = C = O \qquad (VI)$$

in welcher

Ar die oben angegebene Bedeutung hat,

in Gegenwart eines inerten organischen Lösungsmittels, wie beispielsweise Toluol, bei Temperaturen zwischen 20 und 80°C umsetzt (vergleiche hierzu auch die Angaben in Tetrahedron Letters 1965, 1491; Chemische Berichte 111, 3460 (1978), sowie die Herstellungsbeispiele). Die so erhaltenen substituierten Schwefeldiimide der Formel (II) können direkt oder nach Isolierung erfindungsgemäß umgesetzt werden.

Die aliphatischen Schwefeldiimide der Formel (V) sind teilweise bekannt (vergleiche die oben genannten Literaturstellen sowie Chemische Berichte 103, 2152 (1970) und die dort zitierten Literaturstellen), bzw. lassen sie sich nach den dort beschriebenen Verfahren erhalten, indem man Schwefeltetrahalogenide, wie beispielsweise Schwefeltetrachlorid, mit entsprechenden Alkylaminen bei tiefen Temperaturen umsetzt oder indem man Bis(dimethylamino)sulfan auf ein Gemisch des erwähnten primären Amins mit seiner Mono-N-halogenverbindung, insbesondere seiner Mono-N-chlor- oder brom-verbindungen, bei Temperaturen zwischen -30 und +10°C in Chloroform einwirken läßt.

Die Isocyanate der Formel (VI) sind allgemein bekannte Verbindungen der organischen Chemie.

Das weiterhin für das erfindungsgemäße Verfahren als Ausgangsstoff zu verwendende Oxalylchlorid ist durch die Formel (III) definiert. Oxalylchlorid ist eine allgemein bekannte Verbindung der organische Chemie.

Für die erfindungsgemäße Umsetzung kommen als Verdünnungsmittel vorzugsweise inerte organische Lösungsmittel infrage. Hierzu gehören vorzugsweise aromatische Kohlenwasserstoffe, wie beispielsweise Benzol, Toluol, Xylol oder 1,2-Dichlorbenzol, sowie insbesondere aliphatische, halogenierte Kohlenwasserstoffe, wie beispielsweise Methylenchlorid, Chloroform oder Tetrachlorkohlenstoff.

Die Reaktionstemperaturen können bei der erfindungsgemäßen Umsetzung in einem größeren Bereich variiert werden. Im allgemeinen arbeitet man zwischen 0 und 100°C, vorzugsweise zwischen 20 und 60°C.

Bei der Durchführung der erfindungsgemäßen Umsetzung arbeitet man vorzugsweise in molaren Mengen. Die als Zwischenprodukte auftretenden 1,1-Dichlor-3,4-dioxo-1,2,5-thiadiazolidine der Formel (IV) werden ohne Isolierung direkt weiter umgesetzt. Die Isolierung der Verbindungen der Formel (I) erfolgt nach üblichen Methoden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Botrytis-Arten, wie gegen den Erreger des Grauschimmels (Botrytis cinerea), verwendet werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beispiel A

Botrytis-Test (Bohnen) / protektiv

Lösungsmittel: 4,7 Gewichtsteile Aceton

Dispergiermittel: 0,3 Gewichtsteile Alkyl-arylpolyglykolether

Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man Pflanzen von Phaseolus vulgaris im 2-Blattstadium bis zur Tropfnässe. Nach 24 Stunden werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern bonitiert.
Die erhaltenen Boniturwerte werden auf Prozent Befall umgerechnet. 0% bedeutet keinen Befall, 100% bedeutet, daß der Befallsfleck vollständig ausgebildet ist.

Bei diesem Test zeigen z.B. die folgenden Verbindungen eine sehr gute Wirkung, die derjenigen der aus dem Stand der Technik bekannten Verbindungen überlegen ist:

Verbindungen gemäß Herstellungsbeispielen 2 und 3.

Beispiel B

Myzelwachstums-Test

Verwendeter Nährboden:

20 Gewichtsteile Agar-Agar
200 Gewichtsteile Kartoffeldekokt
5 Gewichtsteile Malz
15 Gewichtsteile Dextrose
5 Gewichtsteile Pepton
2 Gewichtsteile Dinatriumhydrogenphosphat
0,3 Gewichtsteile Calciumnitrat

Verhältnis von Lösungsmittelgemisch zum Nährboden:

2 Gewichtsteile Lösungsmittelgemisch
100 Gewichtsteile Agarnährboden

Zusammensetzung Lösungsmittelgemisch

0,19 Gewichtsteile Dimethylformamid oder Aceton
0,01 Gewichtsteile Emulgator Alkylaryl-polyglykoläther
1,80 Gewichtsteile Wasser
2 Gewichtsteile Lösungsmittelgemisch

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten beimpft und bei etwa 21°C inkubiert.

Die Auswertung erfolgt je nach der Wachstumsgeschwindigkeit der Pilze nach 4 - 10 Tagen. Bei der Auswertung wird das radiale Myzelwachstum auf den behandelten Nährböden mit dem Wachstum auf dem Kontrollnährboden verglichen. Die Bonitierung des Pilzwachstums geschieht mit folgenden Kennzahlen:

1 kein Pilzwachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Bei diesem Test zeigen z.B. die folgenden Verbindungen eine sehr gute Wirkung, die derjenigen der aus dem Stand der Technik bekannte Verbindungen überlegen ist: Verbindungen gemäß Herstellungsbeispielen 2 und 3.
In dem Test wurden die folgenden Pilzarten verwendet: Sclerotinia sclerotiorum, Fusarium nivale, Rhizoctonia solani, Cochliobolus miyabenaus, Botrytis cinerea, Pyriculariae oryzae.

Herstellungsbeispiele

Beispiel 1

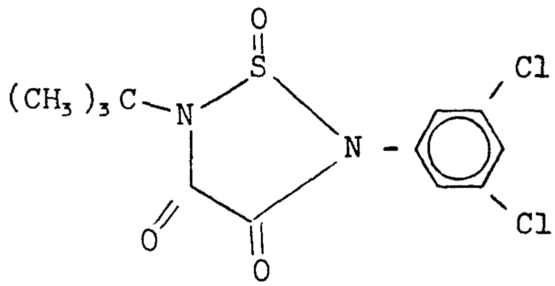

6,3g (0,05 Mol) Oxalylchlorid in 50 ml Tetrachlorkohlenstoff werden unter Rühren zu 13,1g (0,05 Mol) N-3,5-Dichlorphenyl-N'-tert.-butyl-schwefeldiimid in 200 ml Tetrachlorkohlenstoff getropft. Unter Feuchtigkeitsausschluß läßt man 30 Minuten nachrühren, versetzt mit 20 ml Wasser und rührt 2 weitere Stunden. Das ausgefallene Produkt wird abgesaugt, mit Wasser gewaschen und in 300 ml Toluol 3 Stunden am Wasserabscheider erhitzt. Die nach dem Abkühlen ausgefallenen Kristalle werden abgesaugt und getrocknet. Man erhält 12g (72 % der Theorie) 2-(3,5-Dichlorphenyl)-5-tert.-butyl-3,4-dioxo-1,2,5-thiadiazolidin-1-oxid vom Schmelzpunkt 182°C.

Beispiel 2

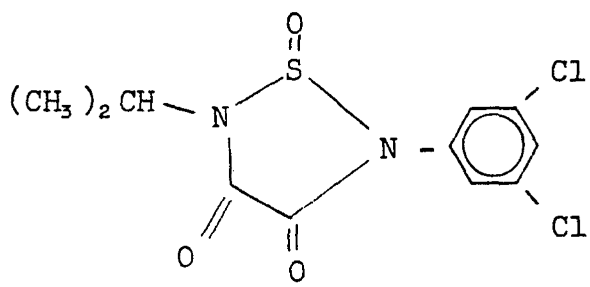

Bei der nachfolgend beschriebenen Arbeitsweise wird zunächst das als Vorprodukt benötigte N-Isopropyl-N'-3,5-dichlorphenyl-schwefeldiimid aus Diisopropyl-schwefeldiimid und 3,5-Dichlorphenylisocyanat dargestellt und anschließend ohne Isolierung mit Oxalylchlorid umgesetzt.

30g (0,2 Mol) Diisopropylschwefeldiimid werden bei Raumtemperatur zu einer Lösung von 37g (0,2 Mol) 3,5-Dichlorphenylisocyanat in 400 ml Toluol getropft. Die Reaktionstemperatur wird zwischen 40°C und 50°C gehalten. Man läßt dann bis zum Erreichen der Reaktionstemperatur nachrühren, engt ein und nimmt den Rückstand in 500 ml absolutem Tetrachlorkohlenstoff auf. Danach werden 25g (0,2 Mol) Oxalylchlorid so zugetropft, daß die Reaktionstemperatur 40°C nicht übersteigt. Eine Stunden nach beendeter Zugabe wird die Reaktionsmischung mit 20 ml Wasser versetzt und bis zum Abklingen der Chlorwasserstoffentwicklung gerührt. Anschließend wird eingeengt, der Rückstand mit Petrolether ausgekocht und die ausfallenden Kristalle abgesaugt. Man erhält 20g (32 % der Theorie) 2-(3,5-Dichlorphenyl)-5-isopropyl-3,4-dioxo-1,2,5-thiadiazolidin-1-oxid vom Schmelzpunkt 109°C.

In analoger Weise werden die folgenden Verbindungen der allgemeinen Formel

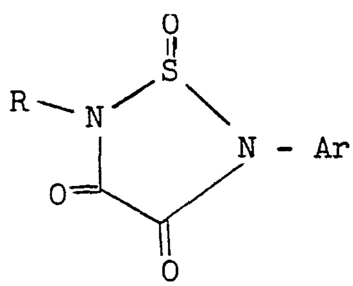

(I)

erhalten:

| Bsp.Nr. | Ar | R | Schmelzpunkt(°C) |
|---|---|---|---|
| 3 | 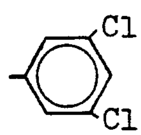 | n-$C_3H_7$ | 126 - 28 |

| Bsp.Nr. | Ar | R | Schmelzpunkt (°C) |
|---|---|---|---|
| 4 | 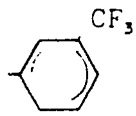 | $C(CH_3)_3$ | 131-33 |
| 5 | 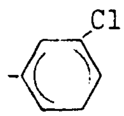 | $C(CH_3)_3$ | 112-15 |
| 6 | 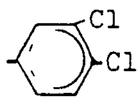 | $C(CH_3)_3$ | 155 |
| 7 | 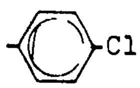 | $C(CH_3)_3$ | 113 |
| 8 | 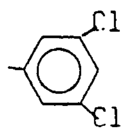 | $C_2H_5$ | 95 - 110 |

Patentansprüche

1. 3,4-Dioxo-1,2,5-thiadiazolidin-1-oxide der allgemeinen Formel

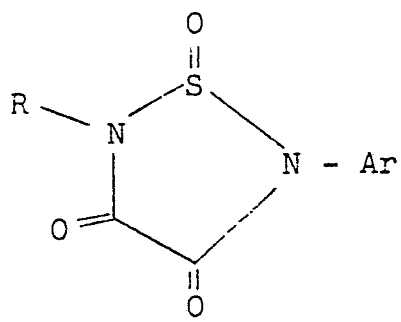

(I)

in welcher

Ar für gegebenenfalls substituiertes Aryl steht und

R für Alkyl steht.

2. Verfahren zur Herstellung von 3,4-Dioxo-1,2,5-thiadiazolidin-1-oxiden, dadurch gekennzeichnet, daß man substituierte Schwefeldiimide der Formel

$$R - N = S = N - Ar \qquad (II)$$

in welcher

Ar und R die oben angegebene Bedeutung haben,

mit Oxalylchlorid der Formel

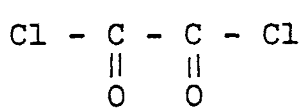

(III)

in Gegenwart eines organischen Verdünnungsmittels umsetzt und die dabei zunächst entstehenden 1,1-Dichlor-3,4-dioxo-1,2,5-thiadiazolidine der Formel

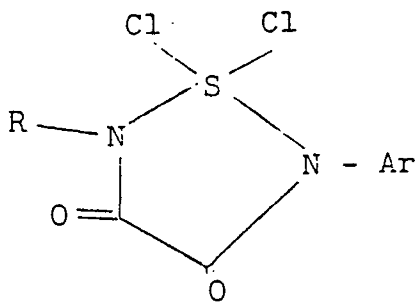

(IV)

in welcher

Ar und R die oben angegebene Bedeutung haben,

ohne deren Isolierung in üblicher Weise zu den entsprechenden 3,4-Dioxo-1,2,5-thiadiazolidin-1-oxiden hydrolysiert.

3. Fungizide Mittel, gekennzeichnet durch einen Gehalt an mindestens einem 3,4-Dioxo-1,2,5-thiadiazolidin-1-oxid gemäß Anspruch 1.

4. Verfahren zur Bekämpfung von Pilzen, dadurch gekennzeichnet, daß man 3,4-Dioxo-1,2,5-thiadiazolidin-1-oxide gemäß Anspruch 1 auf Pilze oder ihren Lebensraum einwirken läßt.

5. Verwendung von 3,4-Dioxo-1,2,5-thiadiazolidin-1-oxiden gemäß Anspruch 1 zur Bekämpfung von Pilzen.

6. Verfahren zur Herstellung von fungiziden Mitteln, dadurch gekennzeichnet, daß man 3,4-Dioxo-1,2,5-thiadiazolidin-1-oxide gemäß Anspruch 1 mit Streckmitteln und/oder oberflächenaktiven Mitteln vermischt.

0013322

Nummer der Anmeldung

Europäisches Patentamt

# EUROPÄISCHER RECHERCHENBERICHT

EP 79104681.6

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | US - A - 3 186 998 (WRIGHT)<br>+ Spalte 1 +<br>-- | 1 | C 07 D 285/10<br>A 01 N 43/82 |
| | US - A - 3 491 109 (ZUMACH)<br>+ Spalten 1,2 +<br>-- | 1 | |
| | US - A - 4 032 533 (SCRIBNER)<br>+ Spalten 1,2 +<br>-- | 1 | |
| | US - A - 4 094 986 (ROKACH)<br>+ Spalten 1,3,4 +<br>---- | 1,3-6 | RECHERCHIERTE SACHGEBIETE (Int. Cl. 3)<br>C 07 D 285/00<br>A 01 N 43/00 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| WIEN | 08-02-1980 | HERING |